# EUROPEAN PATENT APPLICATION

(11) **EP 4 464 302 A1**
(43) Date of publication of application: **20.11.2024**
(21) Application number: 22920450.8
(22) Date of filing: 17.11.2022
(51) Int. Cl.: A61K 6/60, A61K 6/15, A61P 1/02

(54) **POWDER-LIQUID-TYPE DENTURE BASE LINER**

(30) Priority: 13.01.2022 JP 2022003569
(71) Applicant: Tokuyama Dental Corporation, Tokyo 110-0016 (JP)
(72) Inventor: HONDA, Yoshiki, Tokyo 110-0016 (JP); SINAGAWA, Yusaku, Tokyo 110-0016 (JP); YAMAZAKI, Tatsuya, Tokyo 110-0016 (JP)
(74) Representative: Lorenz & Kollegen
(86) International application number: PCT/JP2022/042706
(87) International publication number: WO 2023/135930

(57) **Abstract**

[Object] To exhibit a change in viscosity over time that is capable of ensuring the plastic deformation state necessary for a shaping operation and shorten the curing time by suppressing heat generation in a powder-liquid-type dental plate liner, including: a powder material that includes a non-crosslinked resin powder and a polymerization initiator as main components; and a liquid material that includes a radically polymerizable monomer as a main component, the powder-liquid-type dental plate liner using a roomtemperature-curing-type chemical polymerization initiator that combines a tertiary amine compound and an organic peroxide, one of substituent groups of the tertiary amine compound being an aryl group.

[Solving Means] As the tertiary amine compound, a compound in which two hydrogen atoms of an amine are each substituted with a methyl group and a remaining hydrogen atom is substituted with an aryl group that has a "hydrocarbon group having 1 to 6 carbon atoms" or a "linear hydroxyalkyl group having 1 to 5 carbon atoms and an OH group at a terminal" at a p-position.

## Description

### Technical Field

The present invention relates to a powder-liquid-type dental plate liner that generates low heat and starts curing quickly.

### Background Art

A powder-liquid-type dental plate liner is a material for repairing dentures that have become incompatible with the patient's oral mucosa due to long-term use to make the dentures be in a usable state again. In general, the powder-liquid-type dental plate liner includes a liquid material that contains a radically polymerizable monomer as a main component and a powder material that contains a non-crosslinked resin soluble in the liquid material, and has a mechanism in which the radical polymerization initiator acts after both of the materials are mixed. Note that as the radical polymerization initiator, a chemical polymerization initiator (room temperature redox initiator), a photopolymerization initiator, or a thermal polymerization initiator can be used.

Among these powder-liquid-type dental plate liners, regarding dental plate liners for a "direct method", which are used in a method of directly inserting the dental plate liner into the patient's oral cavity, ensuring compatibility with the oral mucosal surface, and then polymerizing and curing the dental plate liner while holding it in the oral cavity for repairing, they are desired to (1) be of a so-called room temperature curing type that can be cured at room temperature without the need for light irradiation and (2) show a moderate viscosity change over time, i.e., it is not cured immediately when a powder material and a liquid material are mixed, has high fluidity immediately after the mixing, starts curing after the radically polymerizable monomer component penetrates into the non-crosslinked resin component and swelling and dissolution of the component progress until the viscosity increases to the point where plastic deformation occurs, and then enters a state in which plastic deformation does not occur. As a radical polymerization initiator that can meet such demands, a chemical polymerization initiator that combines a tertiary amine compound in which one of substituent groups is an aryl group, such as N,N'-dimethylaniline, N,N'-diethyl-p-toluidine, N-methyl-N'-β-hydroxyethylaniline, and p-tolyldiethanolamine, and an organic peroxide has been known (see Patent Literatures 1, 2, and 3). Among these, as the above tertiary amine compound to be combined with the organic peroxide, N,N-diethyl-p-toluidine (hereinafter, abbreviated as "PEAT" in some cases.) and p-tolyldiethanolamine (hereinafter, abbreviated as "DEPT" in some cases.) represented by the following general formulae are generally used (see Patent Literatures 1 to 4 and Non-Patent Literature 1) because they have high polymerization activity, low irritation, and low odor and can be preserved for a long time while being mixed with the radically polymerizable monomer.

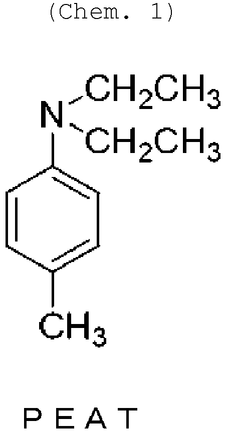

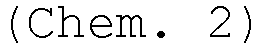

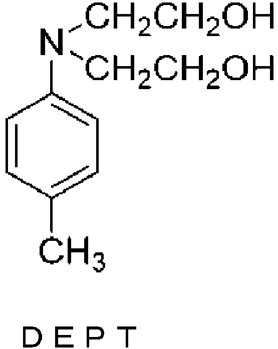

### Citation List

### Patent Literature

Patent Literature 1: Japanese Patent No. 3967488
Patent Literature 2: WO 2002/045660
Patent Literature 3: WO 2018/016602
Patent Literature 4: Japanese Patent Application Laid-open No. 2014-223237

### Non-Patent Literature

Non-Patent Literature 1: The Journal of the Japanese Society for Dental Materials and Devices, vol.21 No.1 62-71 2002

### Disclosure of Invention

### Technical Problem

The powder-liquid-type dental plate liner for a direct method using a chemical polymerization catalyst that combines an organic peroxide and N,N-diethyl-p-toluidine (PEAT) or p-tolyldiethanolamine (DEPT) is an excellent one having the features as described above, but there is a demand for further shortening of the curing time with the aging of patients and the like. That is, in the direct method, since a patient waits for curing in the posture instructed by a surgeon (e.g., chewing or with the mouth open) from the time of performing an operation of inserting the dental plate liner in the patient's oral cavity and shaping it to when curing is finished, there is a problem that waiting in a fixed posture until the curing is finished is extremely burdensome particularly for elderly people whose muscles have weakened. In addition, there is also a need to reduce the heat generation caused by radical polymerization during treatment and reduce patient discomfort during treatment.

The present inventors have tried increasing the amount of PEAT blended in order to meet the above new demand, and the shortening of the curing time has been insufficient although the heat generation caused by radical polymerization has not increased. Further, the present inventors have tried increasing the amount of DEPT blended and found that the heat generation caused by radical polymerization increases and there is a possibility that not only patient discomfort but also burns are caused in some cases although the curing time can be shortened.

In this regard, it is an object of the present invention to provide a powder-liquid-type dental plate liner that exhibits a change in viscosity over time that is capable of ensuring the plastic deformation state necessary for a shaping operation, can be polymerized at room temperature, and is capable of shortening the curing time by suppressing heat generation caused by polymerization and curing, as a powder-liquid-type dental plate liner that can be used suitably for the direct method.

### Solution to Problem

The present invention solves the above problem, and a first embodiment of the present invention is a powder-liquid-type dental plate liner that includes a powder material that includes non-crosslinked resin particles and an organic peroxide and a liquid material that includes a radically polymerizable monomer and a tertiary amine compound, one of substituent groups of the tertiary amine compound being an aryl group (hereinafter, referred to also as "monoaryl tertiary amine".), the powder-liquid-type dental plate liner being characterized in that the tertiary amine compound (monoaryl tertiary amine) includes a compound represented by the following general formula (1). 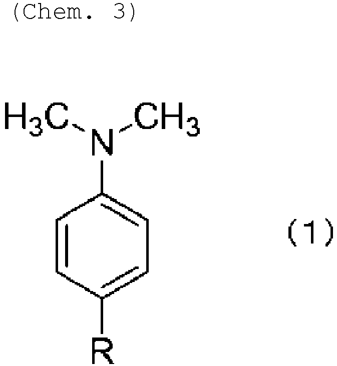 (wherein R represents a hydrocarbon group having 1 to 6 carbon atoms or a linear hydroxyalkyl group having 1 to 5 carbon atoms and an OH group at a terminal.)

In the powder-liquid-type dental plate liner according to the above embodiment (hereinafter, referred to also as the "powder-liquid-type dental plate liner according to the present invention".), the content of the non-crosslinked resin particles, the organic peroxide, and the compound represented by the general formula (1) in the mixture of the powder material and the liquid material with respect to 100 parts by mass of the radically polymerizable monomer is favorably 30 to 450 parts by mass: the non-crosslinked resin particles, 0.2 to 10 parts by mass: the organic peroxide, and 0.1 to 5 parts by mass: the general formula (1), respectively.

Further, it is particularly favorable that the organic peroxide is benzoyl peroxide and the compound represented by the general formula (1) is 2-[4-(dimethylamino)phenyl]ethane-1-ol in which R in the general formula (1) represents 4-tert-butyl-N,N-dimethylaniline that is a t-butyl group or R in the general formula (1) represents a hydroxyethyl group. Further, the powder-liquid-type dental plate liner is favorably a dental plate liner for a direct method that is polymerized and cured directly in a patient's oral cavity.

### Advantageous Effects of Invention

According to the present invention, as a powder-liquid-type dental plate liner that can be used suitably for the direct method, a powder-liquid-type dental plate liner that can be polymerized at room temperature, does not impair the viscosity change properties over time, and has the shortened curing time while suppressing heat generation caused by polymerization and curing is provided.

### Mode(s) for Carrying Out the Invention

### 1. Outline of powder-liquid-type dental plate liner according to present invention

The powder-liquid-type dental plate liner according to the present invention includes a powder material that includes non-crosslinked resin particles and an organic peroxide and a liquid material that includes a radically polymerizable monomer, and a monoaryl tertiary amine, similarly to an existing powder-liquid-type dental plate liner (for a direct method). It is characterized in that the compound represented by the general formula (1) is used as the monoaryl tertiary amine.

Then, in the powder-liquid-type dental plate liner using the above characteristics, i.e., a chemical polymerization initiator that combines an organic peroxide and a monoaryl tertiary amine (hereinafter, referred to also as "organic peroxide/monoaryl tertiary amine".), the above problem is solved by using a specific compound as the above monoaryl tertiary amine.

The reason why such an effect was achieved is not clear, but the present inventors think as follows.

First, the shortening of the polymerization time will be described. In the organic peroxide/monoaryl tertiary amine chemical polymerization initiator according to the present invention, first, an intermediate of a radical active species is generated by the nucleophilic reaction of the lone pair of electrons on the nitrogen atom with the organic peroxide in the monoaryl tertiary amine represented by the general formula (1). That is, in the monoaryl tertiary amine, in the case where the aromatic ring has an electron-donating substituent group at a p-position (4-position), the electron density on the nitrogen atom increases and the nucleophilic reaction proceeds quickly. Subsequently, the hydrogen at the α-carbon (i.e., the methyl group in the general formula (1)) of the alkyl chain substituted with a nitrogen atom of the intermediate is abstracted, thereby generating a radical active species. Further, the above radical active species generated in this way is much more unstable than when using a monoaryl tertiary amine in which a hydrocarbon chain having more carbon atoms than the methyl group is bonded to a nitrogen atom. For this reason, it is conceivable that a polymerization reaction is initiated quickly in the organic peroxide/monoaryl tertiary amine chemical polymerization initiator according to the present invention.

Next, the fact that curing heat generation in radical polymerization is relatively low will be described. This is presumably because that in the organic peroxide/monoaryl tertiary amine chemical polymerization initiator according to the present invention, the monoaryl tertiary amine represented by the general formula (1) and the organic peroxide react with each other and the reaction heat generated when the radical active species is generated is low.

Other than the above features, the components other than the monoaryl tertiary amine, their blending amounts, usages methods, and the like are not particularly different from those in the above existing powder-liquid-type dental plate liner. Hereinafter, the powder-liquid-type dental plate liner according to the present invention will be described, including these points.

Note that in the present specification, the notation "x to y" using numerical values x and y means "x or more and y or less" unless otherwise specified. In such a notation, in the case where a unit is attached to only the numerical value y, the unit applies also to the numerical value x. In the present specification, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

### 2. Powder material and liquid material

The powder-liquid-type dental plate liner according to the present invention is a material that includes a powder material and a liquid material and is used by mixing both of the materials to prepare a dental plate liner (for a direct method). Here, the powder material is a material that is a powdered agent, and the main component thereof is specifically 80 mass% or more, favorably 90 mass% or more of non-crosslinked resin particles. Meanwhile, the liquid material is a material that is a liquid agent, and the main component thereof is specifically 80 mass% or more, favorably 90 mass% or more of a radically polymerizable monomer.

The method of producing the powder material and the liquid material is not particularly limited. For example, predetermined amounts of each component to be blended only need to be weighed out and mixed until uniform states are achieved. Further, the produced powder material and liquid material only need to be preserved in respective containers and may be divided into arbitrary amounts and preserved. Alternatively, the powder material and the liquid material may be weighed out for one use and preserved separately, or they may be separately preserved in a container in which a powder material and a liquid material are housed in the same package.

Each component used in these materials and its blending amount will be described below. Note that since the mixture of the powder material and the liquid material is a dental plate liner that is actually used and the amount of each component contained in these materials is determined with reference to the above mixture, the amount of each component will be described with reference to the amount of the radically polymerizable monomer in the above mixture in the present specification.

### 3. Non-crosslinked resin particles

The non-crosslinked resin particles constitute the main component of the powder material in the powder-liquid-type dental plate liner according to the present invention, non-crosslinked resin particles that are soluble in the radically polymerizable monomer that is the main component of the liquid material are used, similarly to the existing powder-liquid-type dental plate liner. That is, when the powder material and the liquid material are mixed, at least some of such non-crosslinked resin particles are dissolved in the liquid material, the particles of the dissolution residue swell to increase the viscosity of the mixture, and the polymerization of the radically polymerizable monomer is promoted. In addition, the particles of the dissolution residue of this component also have the effect of increasing the toughness of the cured body of the dental plate liner.

Here, the non-crosslinked resin particles that are soluble in the radically polymerizable monomer refer to those that can be dissolved in the amount of 10 parts by mass or more in the radically polymerizable monomer when 200 parts by mass of the non-crosslinked resin particles are mixed with 100 parts by mass of the radically polymerizable monomer at 23°C and stirred.

As a non-crosslinked resin forming such non-crosslinked resin particles that are soluble in the radically polymerizable monomer, (meth)acrylates such as polymethylmethacrylate, polyethylmethacrylate, and a copolymer of methylmethacrylate and ethylmethacrylate, polyethylene, polypropylene, polyamides, polyesters, polystyrenes, or the like can be used. From the viewpoint of high toughness of the cured body, it is favorable to use a polymer of a lower (the alkyl chain has four or less carbon atoms) alkyl(meth)acrylate polymerizable monomer, such as polymethylmethacrylate, polyethylmethacrylate, and a copolymer of methylmethacrylate and ethylmethacrylate.

The average particle diameter of these non-crosslinked resin particles obtained from the D50 value of volume% measured using a laser diffraction/scattering particle diameter distribution apparatus based on the Mie scattering theory is not particularly limited, but is favorably 200 um or less, particularly favorably 1 to 100 µm, considering the compatibility with the radically polymerizable monomer. Note that the shape of the non-crosslinked resin particles is not particularly limited, and may be a spherical shape, an irregular shape, or an amorphous shape.

Further, the weight average molecular weight (standard polystyrene equivalent molecular weight) of suitable non-crosslinked resin particles by a GPC (Gel Permeation Chromatography) method is favorably in the range of 30,000 to 2,000,000, particularly favorably in the range of 50,000 to 1,500,000, considering the mechanical strength of the cured body to be obtained, the solubility in the radically polymerizable monomer component, swelling, and the like.

The blending amount of the non-crosslinked resin particles is favorably in the range of 30 to 450 parts by mass, particularly favorably in the range of 80 to 350 parts by mass, most favorably in the range of 130 to 300 parts by mass, with respect to 100 parts by mass of the radically polymerizable monomer.

### 4. Organic peroxide

The organic peroxide used in the powder-liquid-type dental plate liner according to the present invention functions as a chemical polymerization initiator that generates radicals in coexistence with the compound represented by the general formula (1), and a ketone peroxide, a peroxyketal, a hydroperoxide, a diaryl peroxide, a peroxyester, a diacyl peroxide, a peroxydicarbonate, a hydroperoxide, or the like can be used. Among these, from the viewpoint of preservation stability and ease of availability, a hydroperoxide and a diacyl peroxide are suitable. Examples of particularly suitable organic peroxides include diacyl peroxides such as benzoyl peroxide, 2,4-dichlorobenzoyl peroxide, and lauroyl peroxide, and hydroperoxides such as 1,1,3,3-tetramethylbutylhydroperoxide.

The suitable amount of these organic peroxides to be used differs depending on the type of organic peroxide to be used, and therefore cannot be absolutely determined. However, in general, the suitable amount is within the range of favorably 0.2 to 10 parts by mass, more favorably 0.35 to 7 parts by mass, still more favorably 0.5 to 5 parts by mass, with respect to 100 parts by mass of the radically polymerizable monomer. For example, in the case where benzoyl peroxide or 1,1,3,3-tetramethylbutylhydroperoxide is used, the amount of use is within the range of favorably 0.2 to 10 parts by mass, more favorably 0.4 to 6 parts by mass, still more favorably 0.6 to 4 parts by mass, with respect to 100 parts by mass of the radically polymerizable monomer.

### 5. Radically polymerizable monomer

As the radically polymerizable monomer that is a main component of the liquid material, a radically polymerizable monomer that can be used in the existing powder-liquid-type dental plate liner can be used without particular limitation, and (meth)acrylate polymerizable monomers are suitably used because of their favorable polymerizability and the like. From the viewpoint of high mechanical strength and low irritation materials, it is favorable to contain a radically polymerizable monomer having a molecular weight of 150 to 700, more suitably 180 to 400.

Examples of the (meth)acrylate polymerizable monomer that can be suitable used include 2-(meth)acryloxyethyl propionate, acetoacetoxyethyl(meth)acrylate, 1,6-hexanediol di(meth)acrylate, 1,9-nonanediol di(meth)acrylate, and trimethylolpropane tri(meth)acrylate. Among these, it is particularly favorable to use 2-(meth)acryloxyethyl propionate, acetoacetoxyethyl(meth)acrylate, or 1,9-nonanediol di(meth)acrylate because of their low odor and low irritation in the oral cavity.

### 6. Monoaryl tertiary amine

In the powder-liquid-type dental plate liner according to the present invention, it is necessary to use, as the monoaryl tertiary amine contained in the liquid material (a tertiary amine compound in which one of substituent groups is an aryl group), the compound represented by the following general formula (1) in which two hydrogen atoms of an amine are each substituted with a methyl group and a remaining hydrogen atom is substituted with an aryl group that has R that is a "hydrocarbon group having 1 to 6 carbon atoms" or a "linear hydroxyalkyl group having 1 to 5 carbon atoms and an OH group at a terminal" at a p-position. Note that the compound represented by the following general formula (1) may be blended as a salt with an organic acid such as hydrochloric acid, phosphoric acid, acetic acid, and propionic acid.

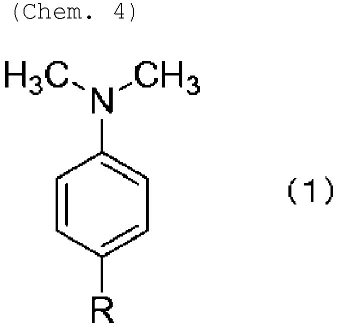

That is, in the case where the N,N-dimethylamino structure is not included and at least one of the two methyl groups bonded to the nitrogen atom is other than a methyl group, or in the case where the aryl group bonded to the nitrogen atom does not include the above group: R only at the p-position even if two methyl groups are bonded to the nitrogen atom, it is not possible shorten the curing time by suppressing polymerization heat generation.

Specific examples of the compound represented by the general formula (1) include N,N-dimethyl-p-toluidine, N,N-dimethyl-4-ethylaniline, N,N-dimethyl-4-propylaniline, N,N-dimethyl-4-isopropylaniline, N,N-dimethyl-4-butylaniline, N,N-dimethyl-4-isobutylaniline, N,N-dimethyl-4-sec-butylaniline, N,N-dimethyl-4-t-butylaniline, N,N-dimethyl-4-pentylaniline, N,N-dimethyl-4-hexylaniline, [4-(dimethylamino)phenyl]methanol, 2-[4-(dimethylamino)phenyl]ethane-1-ol, 3-[4-(dimethylamino)phenyl]propane-1-ol, 4-[4-(dimethylamino)phenyl]butane-1-ol, and 5-[4-(dimethylamino)phenyl]pentane-1-ol.

Note that in the case where the above R represents a hydrocarbon group having 1 to 6 carbon atoms, it is favorably an alkyl group having 1 to 4 carbon atoms from the viewpoint of ease of availability and the like. Further, in the case where the R represents a linear hydroxyalkyl group having 1 to 5 carbon atoms and an OH group at a terminal, it is favorably a hydroxyethyl group or a hydroxypropyl group because it has low volatility and high preservation stability. The following 4-tert-butyl-N,N-dimethylaniline (hereinafter, abbreviated as "tert-DMBA" in some cases.) or 2-[4-(dimethylamino)phenyl]ethane-1-ol {another name: 4-(dimethylamino)phenethyl alcohol} (hereinafter, abbreviated as "DAPE" in some cases.) is particularly favorable. Further, in the case where the compound represented by the general formula (1) is tert-DMBA or DAPE, it is favorable to use benzoyl peroxide as the above organic peroxide.

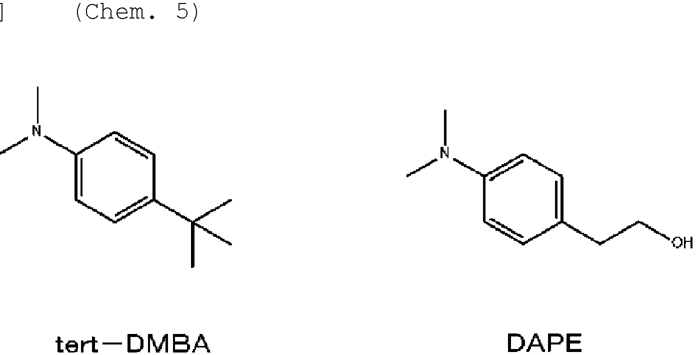

The amount of the compound represented by the general formula (1) to be used is within the range of usually 0.1 to 5 parts by mass, favorably 0.2 to 3 parts by mass, particularly 0.3 to 2 parts by mass, with respect to 100 parts by mass of the radically polymerizable monomer. Further, the amount of the compound represented by the general formula (1) with respect to the organic peroxide is usually 0.03 to 4, favorably 0.08 to 2, particularly favorably 0.15 to 1.5, expressed as a mass ratio to the organic peroxide.

Note that a monoaryl tertiary amine other than the compound represented by the general formula (1), e.g., N,N-diethyl-p-toluidine, N,N-dipropyl-p-toluidine, p-tolyldiethanolamine, or p-tolyldipropanolamine may be blended as long as they do not impar the effects of the present invention.

### 7. Other components

In addition to the above components, a combination of a pyrimidinetrione derivative and an organometallic compound may be blended as a pyrimidinetrione polymerization initiator in the powder-liquid-type dental plate liner according to the present invention in order to improve the curability as long as they do not impair the effects of the present invention.

As the pyrimidinetrione derivative, 1-cyclohexyl-5-methylpyrimidinetrione, 1-cyclohexyl-5-ethylpyrimidinetrione, 5-butyl-1-cyclohexylpyrimidinetrione, 5-sec-butyl-1-cyclohexylpyrimidinetrione, 1-cyclohexyl-5-hexylpyrimidinetrione, 1-cyclohexyl-5-octylpyrimidinetrione, or 1,5-dicyclohexylpyrimidinetrione can be suitably used. Further, as the organometallic compound, copper (II) acetylacetonate, copper (II) acetate, copper (II) oleate, iron (II) acetylacetonate, or the like can be suitably used.

The amounts of the pyrimidinetrione derivative and the organometallic compound to be blended are usually approximately 0.0018 to 0.2 parts by mass: the pyrimidinetrione derivative and 0.00002 to 0.02 parts by mass: the organometallic compound with respect to 100 parts by mass of the radically polymerizable monomer.

Note that usually, the pyrimidinetrione derivative and the organometallic compound are blended into the powder material. Further, in the case where the pyrimidinetrione derivative is blended, an organic halogen compound such as dilauryldimethylammonium chloride and dilauryldimethylammonium bromide may be blended into the liquid material in the amount of 0.1 parts by mass with respect to 100 parts by mass of the radically polymerizable monomer instead of organometallic compound or in addition to the organometallic compound.

Further, in order to improve the fluidity and control the physical properties and an operability of the resulting cured body, an inorganic filler and/or an organic filler (cross-linked resin particles) may be blended into the powder material, an alcohol or a plasticizer such as ethanol, dibutylphthalate, and dioctylphthalate; a polymerization inhibitor such as butylhydroxytoluene and methoxyhydroquinone; an ultraviolet absorber such as 4-methoxy-2-hydroxybenzophenone and 2-(2-benzotriazole)-p-cresol, and a polymerization regulator such as 2,4-diphenyl-4-methyl-1-pentene may be blended into the liquid material, and a dye, a pigment, a flavoring agent, or the like may be blended into the powder material or the liquid material. In the case where the above inorganic filler and organic filler are blended, the total blending amount is favorably kept to 10 parts by mass or less, particularly favorably 6 parts by mass, with respect to 100 parts by mass of the radically polymerizable monomer.

### 8. Method of using powder-liquid-type dental plate liner according to present invention

The powder-liquid-type dental plate liner according to the present invention is used by mixing the powder material and the liquid material to prepare a dental plate liner. At this time, the mixing ratio of the powder material and the liquid material is not particularly limited, and only needs to be appropriately determined by taking into account the content of the above component contained in each member and the desired usage amount of each component when the liquid material and the powder material are mixed as described above. In general, it is favorable to mix the materials at the ratio of the powder material (g) / the liquid material (ml) = 0.3/1 to 4.5/1, particularly favorably the powder material (g) / the liquid material (ml) = 0.8/1 to 3.5/1, most favorably the powder material (g) / the liquid material (ml) = 1.3/1 to 3/1.

Note that each component in the present specification only needs to be blended in the powder material or the liquid material such that each blending amount is satisfied when the powder material and the liquid material are mixed at the above ratio.

Regarding the mixing of the powder material and the liquid material, the powder material and the liquid material only need to be mixed as appropriate in accordance with the packaging form of the materials and used. As an example thereof, it only needs to weigh out desired amounts of the liquid material and the powder material into a rubber cup or the like immediately before use and knead them into a uniform paste using a kneading stick or a spatula for use.

### Examples

Hereinafter, the present invention will be specifically described by way of Examples, but the present invention is not limited to these experimental items. The abbreviations and titles shown in Examples are as follows.

### 1. Powder raw material

### [Non-crosslinked resin particles]

· PEMA1: spherical polyethylmethacrylate particles (average particle diameter 35 µm, a weight average molecular weight 500,000)
· PEMA2:spherical polyethylmethacrylate particles (average particle diameter 70 µm, a weight average molecular weight 1,000,000)

### [Organic peroxide]

· BPO: benzoyl peroxide.

### [Other components (polymerization inhibitor)]

· BHT: butylhydroxytoluene.

### 2. Liquid raw material

### [Radically polymerizable monomer]

· HPr: 2-(meth)acryloxyethyl propionate (molecular weight 186)
· ND: 1,9-nonanediol di(meth)acrylate (molecular weight 296).

### [Monoaryl tertiary amine]

### <Specific monoaryl tertiary amine: compound represented by the general formula (1)>

· DMBA: N,N-dimethyl-4-butylaniline (molecular weight 177)
· tert-DMBA: N,N-dimethyl-4-t-butylaniline (molecular weight 177)
· DAPE: 2-[(4-dimethylamino)phenyl]ethane-1-ol {another name 4-(dimethylamino)phenethyl alcohol} (molecular weight 165)
· DAPP: 3-[(4-dimethylamino)phenyl]propane-1-ol (molecular weight 179)

### <Non-specific monoaryl tertiary amine: compound other than the compound represented by the general formula (1)>

· DMA: N,N-dimethylaniline (molecular weight 121)
· DEFT: p-tolyldiethanolamine (molecular weight 195)
· PEAT: N,N-diethyl-p-toluidine (molecular weight 163).

### Example 1

A powder material was prepared by mixing PEMA1: 110 g and PEMA2: 90 g, which are non-crosslinked resin particles, and BPO: 1.2 g, which is an organic peroxide, for three hours using an oscillating mixer, and a liquid material was prepared by stirring and mixing HPr: 60 g and ND: 40 g, which are radically polymerizable monomers, DMBA: 1.1 g (6.0 mmol), which is the compound represented by the general formula (1) as a monoaryl tertiary amine, and BHT: 0.01 g as another component (polymerization inhibitor), for three hours.

Subsequently, a dental plate liner was prepared by mixing the obtained powder material and the obtained liquid material at the ratio of the powder material (g) / the liquid material (ml) = 2/1, and the curing time and curing heat generation of the dental plate liner were evaluated by the method shown below. Note that the blending ratio of each component in the mixture (dental plate liner) is PEMA1: 110 parts by mass, PEMA2: 90 parts by mass, BPO: 1.2 parts by mass, and DMBA: 1.1 parts by mass (6.0 mmol) with respect to 100 parts by mass of the radically polymerizable monomer (mixture of HPr/HPr mass ratio = 60/40).

### (1) Measurement of curing time

A stainless steel ring (having an inner diameter of 60 mm, an outer diameter of 67 mm, and a height of 2 mm) was placed on a glass plate covered with a plastic sheet, and the tip of a thermocouple was disposed to be located in the center of the ring. The powder material and the liquid material were placed in a rubber cup at the ratio of the powder material (g) / the liquid material (ml) = 2/1 and mixed for 20 seconds, and the mixture was poured into the stainless steel ring and pressed together with the plastic sheet and the glass plate. The mixture was placed in a water bath at 37°C 1 minute and 30 seconds after the start of mixing, and measurement of the temperature of the mixture was started. The curing time defined as the time from the start of mixing until reaching the maximum temperature was 4 minutes and 47 seconds.

Note that clinically, a suitably curing time of the powder-liquid-type dental plate liner is 3 minutes 30 seconds to 10 minutes. When it is shorter than 3 minutes 30 seconds, it is no possible to secure enough time for a series of denture repair operations such as an operation of mixing the powder material and the liquid material, an operation of applying a paste to a denture, a shaping operation in the oral cavity, and removing and trimming operations from the outside of the oral cavity. Further, when it is longer than 10 minutes, the time during which it is inserted in the patient's oral cavity is long, which increases the burden on the patient. Further, in order to reduce the burden on elderly patients, the curing time is favorably shorter in some cases. In this case, the curing time is favorably 3 minutes 30 seconds to 6 minutes.

### (2) Measurement of curing heat generation

The curing heat generation defined as the maximum temperature during the curing time in the above (1) was 45°C.

Note that in the case where the hard dental plate liner is used directly in the oral cavity, the curing heat generation is favorably 37 to 47°C because the higher the curing heat generation, the more painful the patient will feel the irritation caused by the heat generation.

### Examples 2 to 4 and Comparative Examples 1 to 3

Powder-liquid-type dental plate liners were prepared in a way similar to that for Example 1 except that when preparing a liquid material, the type and blending amount of monoaryl tertiary amine to be mixed with 100 parts by mass of the radically polymerizable monomer (mixture of HPr/HPr mass ratio = 60/40) was changed as shown in Table 1, and the curing time and curing heat generation were evaluated. The experimental results are collectively shown in Table 1.

**(Table 1)**

| | Monoaryl tertiary amine | | | | Curing time [min' sec"] | Curing heat generation [°C] |
|---|---|---|---|---|---|---|
| | Classification | Compound | Blending amount | | | |
| | | | mmol | Parts by mass | | |
| Example 1 | Specific | DMBA | 6.0 | 1.1 | 4' 4 7" | 45 |
| Example 2 | | tert-DMBA | 6.0 | 1.1 | 4' 4 2" | 45 |
| Example 3 | | DAPE | 6.0 | 1.0 | 4' 3 7" | 45 |
| Example 4 | | DAPP | 6.0 | 1.1 | 4' 3 2" | 4 5 |
| Comparative Example 1 | Non-specific | DMA | 6.0 | 0.7 | Not cured | - |
| Comparative Example 2 | | DEPT | 6.0 | 1.2 | 5' 1 7" | 5 0 |
| Comparative Example 3 | | PEAT | 6.0 | 1.0 | 8' 3 3" | 4 3 |

Examples 1 to 4 are each an example in which a specific monoaryl tertiary amine, which is the compound represented by the general formula (1), was used as a monoaryl tertiary amine, and each component was blended so as to satisfy the configuration shown in the present invention. In either case, it was shown that the curing time was short and heat generation was low as a dental plate liner.

On the other hand, 1 to 3 are each an example in which a non-specific monoaryl tertiary amine, which does not correspond to the compound represented by the general formula (1), was used as a monoaryl tertiary amine. In Comparative Example 1, since there was no electron-donating substituent group at the p-position (4-position) of the aromatic ring of the monoaryl tertiary amine, the activity of radical polymerization was low and polymerization and curing did not occur. In Comparative Example 2, the curing time was short but the curing heat generation was high. In Comparative Example 3, the curing heat generation was low but the curing time was relatively long.

## Claims

1. A powder-liquid-type dental plate liner that includes a powder material that includes non-crosslinked resin particles and an organic peroxide and a liquid material that includes a radically polymerizable monomer and a tertiary amine compound, one of substituent groups of the tertiary amine compound being an aryl group, the powder-liquid-type dental plate liner being **characterized in that**
the tertiary amine compound includes a compound represented by the following general formula (1). 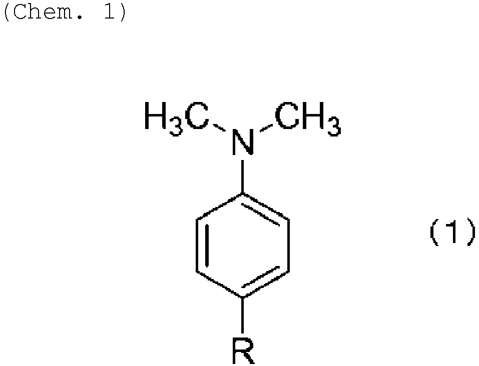 (wherein R represents a hydrocarbon group having 1 to 6 carbon atoms or a linear hydroxyalkyl group having 1 to 5 carbon atoms and an OH group at a terminal.)

2. The powder-liquid-type dental plate liner according to claim 1, wherein
the content of the non-crosslinked resin particles, the organic peroxide, and the compound represented by the general formula (1) in a mixture of the powder material and the liquid material with respect to 100 parts by mass of the radically polymerizable monomer is 30 to 450 parts by mass: the non-crosslinked resin particles, 0.2 to 10 parts by mass: the organic peroxide, and 0.1 to 5 parts by mass: the compound represented by the general formula (1), respectively.

3. The powder-liquid-type dental plate liner according to claim 1 or 2, wherein
the organic peroxide is benzoyl peroxide, and the compound represented by the general formula (1) is 4-tert-butyl-N,N-dimethylaniline or 2-[4-(dimethylamino)phenyl]ethane-1-ol.

4. The powder-liquid-type dental plate liner according to any one of claims 1 to 3, which is a dental plate liner for a direct method that is polymerized and cured directly in a patient's oral cavity.

## Amended claims

### Amended claims under Art. 19.1 PCT

1. A powder-liquid-type dental plate liner that includes a powder material that includes non-crosslinked resin particles and an organic peroxide and a liquid material that includes a radically polymerizable monomer and a tertiary amine compound, one of substituent groups of the tertiary amine compound being an aryl group, the powder-liquid-type dental plate liner being **characterized in that**
the tertiary amine compound includes a compound represented by the following general formula (1), and
the organic peroxide is benzoyl peroxide, and the compound represented by the general formula (1) is 4-tert-butyl-N,N-dimethylaniline or 2-[4-(dimethylamino)phenyl]ethane-1-ol. 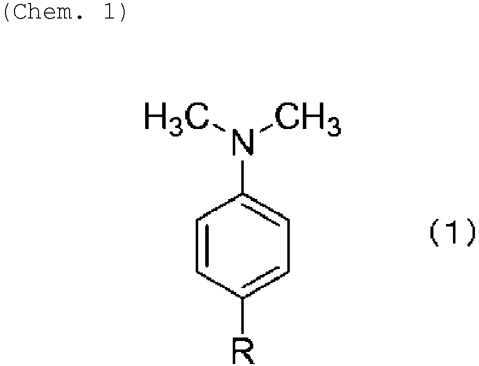 (wherein R represents a hydrocarbon group having 1 to 6 carbon atoms or a linear hydroxyalkyl group having 1 to 5 carbon atoms and an OH group at a terminal.)

2. The powder-liquid-type dental plate liner according to claim 1, wherein
the content of the non-crosslinked resin particles, the organic peroxide, and the compound represented by the general formula (1) in a mixture of the powder material and the liquid material with respect to 100 parts by mass of the radically polymerizable monomer is 30 to 450 parts by mass: the non-crosslinked resin particles, 0.2 to 10 parts by mass: the organic peroxide, and 0.1 to 5 parts by mass: the compound represented by the general formula (1), respectively.

3. The powder-liquid-type dental plate liner according to claim 1 or 2, which is a dental plate liner for a direct method that is polymerized and cured directly in a patient's oral cavity.
